# EUROPEAN PATENT SPECIFICATION

(11) **EP 2 086 507 B1**
(45) Date of publication and mention of the grant of the patent: **07.11.2018**
(21) Application number: 07818736.6
(22) Date of filing: 05.10.2007
(51) Int. Cl.: A61K 9/127, A61K 38/18, A61K 9/19, A61K 35/35

(54) **A SPINAL NUCLEUS PULPOSUS IMPLANT**
GALLERTKERN IMPLANT
IMPLANT DE NOYAU GÉLATINEUX SPINAL

(30) Priority: 06.10.2006 EP 06021093
(43) Date of publication of application: 12.08.2009
(73) Proprietor: BioNet Pharma GmbH, 80331 München (DE)
(72) Inventor: DONY, Carola, 81477 München (DE); HELLERBRAND, Klaus, 82272 Moorenweis (Eismerszell) (DE); HUSTERT, Elisabeth, 82110 Germering (DE); PIPPIG, Susanne, 81377 München (DE); SIGL, Rainer, 82178 Puchheim (DE)
(74) Representative: Weickmann & Weickmann PartmbB
(86) International application number: PCT/EP2007/008660
(87) International publication number: WO 2008/040557

(56) References cited:
- WO-A-00/12762
- WO-A-00/44401
- WO-A-2005/014071
- WO-A-2005/120595
- US-A1- 2004 230 310
- US-A1- 2005 031 666
- RISBUD M V ET AL: "Stem cell regeneration of the nucleus pulposus", THE SPINE JOURNAL, ELSEVIER, AMSTERDAM, NL, vol. 4, no. 6, 1 November 2004 (2004-11-01), pages S348-S353, XP027203622, ISSN: 1529-9430 [retrieved on 2004-11-22]

## Description

The present invention relates to a spinal nucleus pulposus implant for the treatment of the intervertebral disc and, in particular, to the use of a CD-RAP protein therefore.

Degeneration of the intervertebral disc (IVD) is a multifactorial process involving mechanical, genetic and biological factors. Although the pathophysiological mechanism remains unclear, resultant changes in structure and function of the disc have been well described. Unlike articular cartilage, the IVD is composed of different tissues. The healthy IVD is a well-encapsulated, avascular organ which contains a jelly-like nucleus pulposus (NP) surrounded by a fibrous annulus fibrosus (AF), which provides mobility and a cushion between the vertebrae. The nucleus pulposus is located at the center of each disc and is composed of chondrocytes which produce an extracellular matrix containing a high percentage of proteoglycans (PG) and type II collagen in the adult. The nucleus pulposus is surrounded by the annulus fibrosus which consists of highly organized, directionally oriented collagen fibers oriented in concentric lamellae, and extracellular matrix. The inner annulus fibrosus is thicker than the outer and has a fibrocartilaginous matrix that lacks the lamellar structure. A thin distinct region, the transition zone (TZ), divides the inner annulus from the nucleus pulposus.

During the aging process, the reduction in proteoglycan content of the nucleus leads to decreased hydration and evidence of degeneration, including reduction in disc height and increased load on the surrounding structures of the spine. At the biological level it reflects an imbalance between the normal anabolic and catabolic function of the nucleus pulposus cells. In some cases of degenerative disc disease (DDD), gradual degeneration of IVD is caused by mechanical instabilities. Increased load and pressure on the nucleus pulposus cause the cells or invading macrophages to produce larger amounts of cytokines or toxic amounts of metalloproteinases (MMPs). As DDD progresses, toxic levels of cytokines and MMPs degrade the extracellular matrix and lead to a destruction of the proteoglycans, thereby reducing the water-retaining capabilities with resulting dehydration of the nucleus pulposus. In the following the flexibility of the nucleus pulposus is reduced and delamination of the annulus fibrosus might be the consequence, eventually developing internal fissures spreading out towards the periphery. These alterations cause even more mechanical instability and induction of cytokine production, which progress the DDD and the disc begins to bulge (herniated disc disease) and ultimately ruptures, with nerve irritation and low back pain.

Unfortunately, most current therapies for disc-related low back pain are targeted to obtain symptomatic relief rather than repairing the underlying degenerative process. Conservative treatment consists of physical measures, the use of analgetics, muscle relaxants, non-steroidal anti-inflammatory drugs, systemic corticosteroids, epidural injections and injections of cytokine antagonists. In later stages of therapy the treatment of a degenerated disc is either removal of the degenerated disc and fusion of adjacent vertebrae on either side of the disc or a replacement of the disc by a synthetic disc material.

Disc replacement or fusion do not restore normal disc height, physiology or mechanical properties and might lead to further symptoms at either the site of surgery or adjacent discs. Therefore, future treatment methods are needed which inhibit or reverse the cellular disturbances underlying the degeneration and restore the biological function of the vertebral disc. Many researchers worldwide are seeking biological ways to repair degenerated intervertebral discs.

Chemonucleolytic agents, such as chymopapain, have been used in the past as a method of treating herniated IVDs. Pain relief was linked to the ability to degrade PGs, thereby decreasing intradiscal pressure and relieving compression of the affected nerve roots. However, associated complications such as anaphylaxis, neurological injury and infection diminished the use of chymopapain. More recently, chondroitinase ABC (C-ABC) has been suggested as an alternative for chemonucleolysis because it lacks protease activity and possesses a narrower substrate spectrum. Although the regeneration of the cartilaginous matrix occurs earlier after treatment with C-ABC than with chymopapain, disc height and PG content do not recover sufficiently and the IVD is left with altered suboptimal biochemical properties thus accelerating the degenerative cascade of events (Takegami et al., 2005; Masuda et al., 2004; Masuda and An, 2004).

WO2005/000283 and references therein describe methods treating degenerative disc disease including injecting an antagonist such as high affinity antagonists of MMP, highly specific cytokine antagonists, a highly specific p38 kinase inhibitor, anti-inflammatory drugs, a cycline compound, anti-proliferative or anti-apoptotic agents into a diseased intervertebral disc. These compounds are believed to inhibit catabolic processes within the DDD by inhibition of pro-inflammatory cytokines, MMPs, prostaglandin regulation or reduction in pro-inflammatory effects or inhibit chondrocyte proliferation or apoptosis, but no anabolic activity has been described.

WO2006/086105 describes methods for treating and/or reversing disorders of the intervertebral disc using transcription factor inhibitors which target transcription factors such as NF-ₖB, E2F, GATA-3 and STATs.

WO2006/031376 describes a method of treating degenerative disc disease comprising administration of an antioxidant into the intervertebral disc either alone or in combination with an additional therapeutic agent such as fibrin, hyaluronic acid, stem cells, bone marrow, or a growth factor.

In other studies exogenous growth factors like transforming growth factor beta-1 (TGF-β1), insulin like growth factor-1 (IGF-1), bone morphogenetic protein-2 (BMP-2), growth and differentiation factor-5 (GDF-5) and osteogenic protein (OP-1) have been administered to intradiscal cells or injected intradiscally to stimulate synthesis of proteoglycans and collagen to slow down or reverse the degeneration of the IVD (Levicoff et al., 2005; Sobajima et al., 2004; Takegami et al., 2005; Kawakami et al., 2005). However, these growth factors are rather unspecific having the risk to induce osteogenic genes and therefore may induce undesired bone formation. Another limitation of the application of this growth factor technology is the relatively short biological half-life of the exogenous growth factor that would enable only transient biological effects after their delivery.

Therefore, the object of the present invention is to provide a spinal nucleus pulposus implant which improves or restores the biomechanical properties of the vertebral disc and/or inhibits further progression of diseases affecting the vertebral disc.

Another object of the present invention is to provide a nucleus pulposus implant comprising a cartilage differentiation and maintenance factor able to reverse disease processes affecting the vertebral disc, to restore the properties of the vertebral disc or to inhibit further progression of the disease.

Another object of the present invention is to modify cartilage homeostasis by stimulating anabolic processes on the expense of catabolic processes within the IVD for providing a new approach for treating chronic conditions such as DDD.

Another object of the present invention is to provide a nucleus pulposus implant comprising a cartilage differentiation and maintenance factor for treatment of localized degeneration of the vertebral disc, in which the cartilage differentiation and maintenance factor provides the patient with a better opportunity to heal, slow disease progression, and/or otherwise improve patient's health.

Another object is to provide a spinal nucleus pulposus implant to increase the disc height and proteoglycan content in the IVD tissue.

Another object of the present invention is to provide a nucleus pulposus implant comprising a cartilage differentiation and maintenance factor for providing suppression and inhibition of the action of specific cytokines in humans to treat chronic conditions such as DDD in addition to cartilage maintenance.

Another object of the present invention is to provide a spinal nucleus pulposus implant which may be implanted or injected by minimal invasive procedures or endoscopically.

Another object of the present invention is to provide a nucleus pulposus implant delivery system capable of providing more prolonged levels or sustained controlled release of the therapeutic agent ensuring that the agent is available at the site of degenerated IVD for a longer time frame.

Another object of the present invention is to provide a spinal nucleus pulposus implant which is a delivery system capable of providing more prolonged levels of a therapeutic agent.

To achieve the objects of the invention, a spinal nucleus pulposus implant or formulation is provided which comprises a cartilage differentiation and maintenance factor which is a non-antibody or non-receptor molecule. In particular, the present invention provides a spinal nucleus pulposus implant or formulation comprising a cartilage differentiation and maintenance factor comprising the mature sequence of CD-RAP according to Seq. ID No. 1, or amino acids 12 to 107 of Seq. ID No. 1, for use in the treatment of a spinal disorder.The implant, in particular, is injectable or implantable transdiscally. For clarity, non-antibody means that the cartilage differentiation and maintenance factor is not an antibody such as for example a monoclonal, polyclonal or chimeric antibody against TNF alpha. Receptor molecule means a receptor molecule with a high specificity against pro-inflammatory cytokines such as TNFα, truncated forms thereof or functional equivalents thereof.

The present inventors have developed a number of procedures for treating pathological spinal disorders such as degenerative disc disease by application of an effective amount of a functional biomolecule into a pathological spinal disorder (e.g. DDD). In accordance with the present invention, one embodiment encompasses a spinal nucleus pulposus implant or a formulation comprising a cartilage differentiation and maintenance factor, which is transdiscally injectable, preferably by one or repeated injections, or implantable into an intervertebral disc. The cartilage differentiation and maintenance factor is selected from the highly cartilage specific protein CD-RAP or active fragments thereof able to stimulate the differentiation and maintenance of IVD cells while inhibiting undesired bone formation.

The cartilage differentiation and maintenance factor CD-RAP inhibits the activity of cytokines and MMPs and/or reduces the expression of such cytokines and MMPs while stimulating anabolic processes resulting in partial or complete restoration of the degenerated tissue e.g. fibrocartilage and are therefore able to reverse or stop the disease process. Thereby an injection of the cartilage differentiation and maintenance factor assists in arresting the aging process of the degenerating disc. Accordingly, the present invention enables to treat a degenerative disc at an earlier stage and thereby prevents degradation of the extracellular matrix.

In addition, the cartilage differentiation and maintenance factor alleviates or prevents cartilage degradation and preserves or improves the structure and function of the IVD preferably without undesired bone formation.

Another embodiment of the present invention relates to the use of a cartilage determination and maintenance factor for manufacturing of a pharmaceutical composition, which is a spinal nucleous pulposus implant or formulation, for treating a spinal disorder in a mammal in need of such treatment.

In a preferred embodiment the spinal disorder is idiopathic low back pain, disc herniation, internal disc disruption or fissured discs, radiculopathy, spinal stenosis, herniated nucleus pulposus-induced sciatica, sciatica, idiopathic scoliosis or myelopathy.

Yet another aspect of the present invention comprises the use of the spinal nucleus implant or formulation which comprises a cartilage differentiation and maintenance factor comprising the mature sequence of CD-RAP according to Seq. ID No. 1, or amino acids 12 to 107 of Seq. ID No. 1, wherein the implant or formulation is injectable or implantable transdiscally, for manufacturing of a pharmaceutical composition for treating a spinal disorder in a mammal in need of such treatment.

For the purpose of the present invention, the term "spinal nucleus pulposus implant" means a device or a preparation which is to be administered in the intervertebral disc, in particular, into the nucleus pulposus (NP) of the intervertebral disc. Preferably, the implant can be administered directly into the intervertebral disc through the AF or deposited directly into the NP of the disc. It might be injected into the disc through a needle or other means of minimal invasive application. It is also meant that the spinal nucleus pulposus implant is a pharmaceutical preparation which can be injected into the nucleus pulposus, intradiscal space or intervertebral space. The implant can be a solid, e.g. a sponge or a solid carrier, which comprises a cartilage differentiation and maintenance factor. Preferably, the implant is liquid which allows for easy application thereof. The implant, for example, can be a liquid comprising a cartilage differentiation and maintenance factor comprising the mature sequence of CD-RAP according to Seq. ID No. 1, or amino acids 12 to 107 of Seq. ID No. 1, optionally together with other drugs, formulation aids or carriers. In instances of applying a larger size of a carrier material partial or total removement of the disc might be preferred.

For the purpose of the present invention, the term "transdiscal administration" includes but is not limited to injection of the spinal nucleus pulposus implant into an intervertebral disc, in particular, into the NP of an intervertebral disc which includes an intact disc, a degenerated disc of different stages, a herniated disc, a ruptured disc, a delaminated disc or a fissured disc. If the volume to be injected might cause pressure of the NP, at least part of the NP can be removed prior to injection or application of the implant for the spinal column. In some cases the volume of the removed material is about the amount of volume ± 20% to be applied. The term transdiscal administration also includes an injection of the spinal nucleus pulposus implant into the AF of a degenerating or intact disc as described above for the NP. In instances of applying a larger size of a carrier material partial or total removement of the disc might be necessary before application of the spinal nucleus pulposus implant. It further includes providing the implant into a location outside but closely adjacent to the AF wall or endplate of an adjacent vertebral body, this might avoid the puncture of the AF and therefore potential burden on the disc.

The term "degenerative disc disease (DDD)" is a chronic process characterized in part by progressive loss of proteoglycan and water content in the nucleus pulposus that can become manifest in multiple disorders such as idiopathic low back pain, disc herniation, internal disc disruption or fissured discs, radiculopathy, spinal stenosis, herniated nucleus pulposus-induced sciatica, sciatica, idiopathic scoliosis and/or myelopathy. The disc degeneration grade can be ranked by analysis of preoperative MRI.

The term "cartilage differentiation and maintenance factor" means one or more cartilage differentiation factors that might have mitogenic capability but are characterized by their ability to increase and/or maintain the chondrocyte-specific phenotype of the cell (e.g. anabolic activity) without undesired bone formation. Chondrocyte-specific characteristics are for example the production of aggrecan, type II collagen, SOX-9 and proteoglycans. Chondrogenic morphogens may reverse the dedifferentiated or fibrotic phenotype of disc cells to a fibrochondrocytic phenotype comparable to disc nucleus cells of younger and normal adult discs. It may also have an anabolic effect on annulus cells and/or endplate cells of the disc. The cartilage differentiation and maintenance factor is preferably a secreted molecule and hence can potentially act in autocrine, paracrine or endocrine fashion.

"Mesenchymal stem cells (MSCs)" according to the present invention are primitive or resting cell populations that reside in many mature skeletal tissues as uncommitted mesenchymal progenitor cells. MSCs are flexible and have the ability to differentiate towards several mature tissue types, including cartilage, bone, fat and other tissue, depending on the environment and biological signals provided to these resting cells. MSCs are available from many autologous sources, including bone marrow, blood, muscle tissue and fat that can be harvested to isolate these cells without significant donor site morbidity or immunogenic potential.

MSCs can be precursor cells of NP cells or AF cells, chondrocytes, or other living cells that could function like AF or NP cells or could differentiate into cells or build a functional NP and/or AF.

"Treating or treatment" as used herein, means an alleviation of symptoms associated with a disorder or disease, halt of further progression or worsening of the symptoms, prevention or prophylaxis of the disease or disorder.

The invention is based on the finding that a cartilage differentiation and maintenance factor such as a member of the MIA (melanoma inhibitory activity factor) family like CD-RAP can affect or modify a spinal disorder by anabolic effects (e.g. regeneration or restoration of fibrocartilage within the vertebral column) while arresting or inhibiting catabolic degenerative processes (e.g. degeneration of the extracellular matrix) within the spinal disc.

Such an effect of a cartilage differentiation and maintenance factor on spinal disorders is surprising, since the cartilage of the intervertebral disc (IVD) is quite different from other common cartilage.

In contrast to articular cartilage which is referred to hyaline cartilage the cartilage of IVDs consists of fibrocartilage which is a special type of cartilage. Particularly the AF of the IVD is considered fibrocartilaginous and consists primarily of lamellae composed of highly oriented collagen fibers. The NP contains a higher content of type II collagen that is randomly orientated, with a much higher concentration of proteoglycans. Cells of the AF have shown to orientate along the predominant collagen fiber direction of the lamella. Cells of the innermost AF and NP region are more rounded and accumulate more type II and VI collagens and proteoglycans.

There are significant physical and chemical differences between fibrocartilage and other types of cartilage such as articular/hyaline cartilage (WO2005/091960 and references therein). For example fibrocartilage differs in having much type I collagen in its matrix mainly in the annulus. Type II collagen from fibrocartilage such as IVD cartilage, has a substantially higher level of hydroxylation and glycosylation than type II collagen from articular cartilage and aggrecan is more highly substituted. These posttranslational modifications affect the structure and physical function of the fibrocartilage collagen and IVD. Antenatal differentiation of the fibrocartilaginous IVD also differs from that of the articular cartilage in a synovial joint. The IVD has a complex developmental history and contains notocord derived cells which have no equivalence in articular cartilage.

Until the present invention, cartilage differentiation and maintenance factors of the present invention comprising the mature sequence of CD-RAP according to SEQ ID No. 1, or amino acids 12 to 107 of SEQ ID No. 1 had not been shown to have biological effects to prevent fibrocartilage or fibrochondrocytes degeneration *in vitro* or *in vivo.* The cartilage differentiation and maintenance factors of the present invention comprising the mature sequence of CD-RAP according to SEQ ID No. 1, or amino acids 12 to 107 of SEQ ID No. 1 induce and maintain cartilage anabolism in the IVD (e.g. synthesis of proteoglycan and aggrecan) while the pathogenesis of degeneration and cartilage catabolism including breakdown of matrix, synthesis of abnormal proteoglycans and collagens is partially or fully arrested, inhibited or even reversed.

Abnormal disc degeneration, increased apoptosis and diminished matrix molecule synthesis are at least partially mediated by cytokines such as IL-1 which is shown to switch chondrocytes from anabolism to catabolism, inducing cartilage breakdown at molecular and morphological level. Inhibition of the activity of cytokines and MMPs, known to be involved in disc degeneration and/or reduction of the expression of such cytokines and MMPs mediated by the present invention aids in re-synthesis of normal disc matrix and influences discal cell function. Thereby an injection of the cartilage differentiation and maintenance factors assists in arresting or reversing the aging or degenerative process of the degenerating disc. Accordingly, the present invention enables to treat a degenerative disc at an earlier stage and thereby prevents degradation of the extracellular matrix and preserves the structure and function of the IVD.

The cartilage differentiation and maintenance factor of the present invention is a non-antibody and non-receptor molecule. This means that the factor used according to the invention is not an antibody and not a receptor molecule either. The cartilage differentiation and maintenance factor is a non-transcription factor (e.g. is not SOX-9) or is an extracelluar protein. The cartilage differentiation and maintenance factor is not selected from the group of transforming growth factor β (TGF-β e.g. TGF-β1)-, bone morphogenetic (BMP)- and insulin like growth factor (IGF)-family proteins (e.G. IGF-1). BMP proteins are described by Wozney et al. (Wozney and Rosen, 1998) and include for example BMP-2, BMP-7 and growth and differentiation factors such as GDF-5, GDF-6 and GDF-7.

Preferably, the cartilage differentiation and maintenance factor according to the invention which comprises the mature sequence of CD-RAP according to SEQ ID No. 1, or amino acids 12 to 107 of SEQ ID No. 1, is a chondrogenic morphogen, more preferably a chondrogenic morphogen which is a protein, preferably a cartilage specific protein, with anabolic activity for cartilage regeneration (anabolic factor) and maintenance. The anabolic factor in contrast to catabolic factors such as metalloproteinases, apoptotic factors, interleukins, prostaglandins, proteolytic and degradative enzymes, oxygen free radicals, nitric oxide and fibronectin fragments which induce degradation of the nucleus pulposus region, increase the chondrocyte-specific phenotype of cells within the vertebral disc. Preferably, the chondrogenic morphogen is a cartilage determination factor preferably specific for cartilage tissue controlling cartilage formation and maintenance, while avoiding or inhibiting formation of bone.

In one embodiment, the cartilage differentiation factor has a molecular weight of less than 80 kDa, preferably of ≤ 30 kDa, more preferably of ≤ 15 kDa, most preferably between 10 and 15 kDa.

The cartilage differentiation and maintenance factor used according to the invention which comprises the mature sequence of CD-RAP according to SEQ ID No. 1, or amino acids 12 to 107 of SEQ ID No. 1, is a factor which induces the synthesis of extracellular matrix proteins such as proteoglycan, aggrecan and/or collagen. Further, preferably, the factor results in a reduction of the amount of cytokines and MMPs.

The cartilage differentiation and maintenance factor according to the invention which comprises the mature sequence of CD-RAP according to SEQ ID No. 1, or amino acids 12 to 107 of SEQ ID No. 1, is a protein with an SH3-domain or with a domain which adopts an SH3-like domain fold. SH3-domain or SH3-like domain are described for example in Stoll et al. (Stoll et al., 2001b) and can be determined by the prediction of an SH3-fold by an 3D-PSSM Web server published in Kelley et al. (Kelley et al., 2000). SH3-domains also, called Src homology domains, are protein molecules that are found in many intracellular proteins. So far, no SH3-domain proteins were described to be useful in treatment of spinal disorders.

In another embodiment the cartilage differentiation and maintenance factor according to the invention which comprises the mature sequence of CD-RAP according to SEQ ID No. 1, or amino acids 12 to 107 of SEQ ID No. 1, is a protein which specifically can bind to fibronectin, fibronectin fragments and/or proline rich sequences as for example described in the literature (Stoll et al., 2001a; Homandberg and Hui, 1996; Homandberg et al., 1997).

The cartilage differentiation and maintenance factor according to the invention which comprises the mature sequence of CD-RAP according to SEQ ID No. 1, or amino acids 12 to 107 of SEQ ID No. 1, comprises a fibronectin or integrin binding domain. Binding of the cartilage differentiation and maintenance factor to extracellular proteins such as fibronectin or fibronectin fragments as well as integrins can be determined for example by ELISA. Fibronectin, fragments or integrins thereof can be coated on plastic surfaces and are exposed to the cartilage differentiation and maintenance factor. The amount of binding can be determined by a peroxidase-linked monoclonal antibody against the cartilage differentiation and maintenance factor. Integrin binding can also be determined as described by Bauer et al. herewith incorporated by reference.
Mature CD-RAP sequence (SeqID No. 1)
Generic sequence 1 (SeqID No. 02)
   C X₄ C X₁₇ C X₁₂ V X₁₁₋₁₃WX₇₋₁₈ FX₄VX₂₁C X
Generic sequence 2 (SeqID No. 03)
Generic sequence 3 (SeqID No. 04)
wherein "X" at each occurrence independently represents any amino acid and the number in lowercase the number of any amino acid. Preferably, "X" independently represents a naturally occurring amino acid and, in particular, A, R, N, D, B, C, Q, E, Z, G, H, I, L, K, M, F, P, S, T, W, Y or V.

The cartilage differentiation and maintenance factor is CD-RAP (Cartilage derived retinoic acid sensitive protein), also named MIA (melanoma inhibitory activity), OTOR (fibrocyte derived protein, FDP, MIA-like, MIAL) and TANGO 130 which belongs to a class of secreted proteins (Bosserhoff et al., 2004; Bosserhoff and Buettner, 2003; Bosserhoff et al., 1997; WO00/12762). CD-RAP or MIA is a 130 amino acid protein (EP 0710248, EP 1146897) that is a highly specific marker for chondroid differentiation. Gene expression is activated at the beginning of chondrogenesis throughout cartilage development (Dietz and Sandell, 1996; Sakano et al., 1999). In case of cartilage damage due to osteoarthritis, CD-RAP is expressed with increasing levels at the onset of disease at which time a strong anabolic effect is observed and will decline once the disease worsens (Saito et al., 2002).

The protein contemplated herein can be expressed from intact or truncated genomic DNA or cDNA or from synthetic DNAs, in prokaryotic or eukaryotic host cells. Proteins can be isolated from the culture media or inclusion bodies and/or refold to form biological active compositions. See e.g. EP 0710248 and Lougheed et al. (Lougheed et al., 2001) for exemplary protocols for recombinant protein purification of CD-RAP. Detailed description of how to test the activity (e.g. chondrogenesis) of such isolated proteins is described in Tscheudschilsuren et al. and Stoll et al. (Tscheudschilsuren et al., 2005; Stoll et al., 2003). A bioassay for cartilage induction is described in example 2 to 5 in EP1146897. Example 5 describes a mouse ectopic implant assay for cartilage induction. Alternatively cartilage induction and maintenance can be determined in a partial or full thickness articular cartilage repair model.

In one embodiment of the present invention, the spinal nucleus pulposus implant further comprises one or more additional active agents, preferably anticatabolics (e.g. TIMP-1 and TIMP-2), mitogens (e.g. IGF-1, PDGF, EGF, FGF), bone morphogenetic proteins such as GDF-5, BMP antagonists such as noggin or chordin and/or intracellular regulators (e.g. LMP-1, SOX-9) or combinations thereof. The addition of anticatabolics further increases matrix synthesis mediated by the cartilage differentiation and maintenance factor or chondrogenic morphogen for example by inhibition of degradative enzymes. Mitogenic molecules are growth factors which increase the rate of mitosis of cells and might also increase PG synthesis to various degrees depending on the region of the disc where the cells are derived from and therefore further support the effect of the cartilage differentiation and maintenance factor or chondrogenic morphogen. By combining a cartilage differentiation and maintenance factor with an intracellular regulator up-regulation of the cartilage differentiation and maintenance factor and/or PG synthesis can be achieved in *in vitro* experiments.

In another embodiment, the spinal nucleus pulposus implant further comprises one or more anti-metalloproteinases, cycline compounds, cytokine antagonists, TNF inhibitors, IL-inhibitors, anti-angiogenic substances, inhibitors of proteolytic enzymes, anti-inflammatory drugs including infliximab, etanercerpt, adalimulab, nerelimonmab, lenercerpt and the like, or combinations thereof.

In another embodiment, the spinal nucleus pulposus implant is co-administered or administered after injection or application of chemonucleolytic agents such as C-ABC or those described in US 2005/0031666 in order to prevent a long-term structural damage of the disc. Preferably, the spinal nucleus implant is injectable or implantable transdiscally. Preferably, the injection is local or non-systemic injection. An advantage for injection or implantation transdically is that higher concentrations of the cartilage differentiation and maintenance factor can be used while causing minimal systemic toxicity.

The cartilage differentiation and maintenance factor comprising the mature sequence of CD-RAP according to Seq. ID No. 1, or amino acids 12 to 107 of Seq. ID No. 1, can directly be implanted or injected in an acceptable solvent or vehicle, for example, but not limited to physiological saline solution, sterilized water, Ringer's solution. Preferably, it is administered into the intradiscal or NP space. Administration can be achieved with a single or repetitive injection(s), preferably with a percutaneous injection or percutaneously via a catheter. The intradiscal injection of the chondrogenic protein CD-RAP will increase intervertebral disc height by stimulating intervertebral disc cells to upregulate proteogycan, aggrecan and/or collagen synthesis. Clinical application can thus be accomplished by minimal invasive techniques, significantly saving costs and the likelihood of complications relative to other procedures such as partial disectomy or vertebral fusion.

While it is possible to add the spinal nucleus pulposus implant of the invention to the intervertebral disc, it is also possible to remove part of the intervertebral disc and replace it by the implant of the invention.

The present invention, thus, also provides for replacing an amount of the NP removed, for example, in a nucleotomy or partial nucleotomy procedure, or for supplementing a NP that has become degenerated by reason of age, injury or the like with a nucleus pulposus implant of the present invention. The degenerating or degenerated disc or a part thereof may be removed with standard techniques, with a laser, shaver, or other surgical instrument.

The degenerating disc can be an intact disc or a ruptured disc. The degenerating disc can be delaminated, can have fissures or can be herniated.

The spinal nucleus pulposus implant may be combined with a minimal invasive stabilizing procedure. In severe cases of advanced disc degeneration where continuous stimulation results in production of undesired factors such as catabolic factors a minimal invasive stabilizing procedure can further support regeneration or inhibit progression of the degenerated disc.

In one embodiment of the invention, the implant or formulation further comprises a carrier or a drug delivery device. The carrier or drug delivery device used in the invention is biocompatible in that it is not toxic and does not elicit inflammatory reactions in the body. The carrier can include a matrix or scaffold structure. The carrier may be solid, a liquid, a gel, a paste or other injectable form. Preferably, the carrier comprises a hydrogel as for example described in WO2005/113032, in particular injectable hydrogels, sulphated hyaluronic acid, a highly substituted carboxymethylcellulose and salts thereof, alginate, hydroxypropylalginate, chitosan, hydroxethylstarch, collagen, gelatin, reverse thermal gels (e.g. Pluronic F128), a chitosan based thermosensitive copolymer (e.g. chitosan-Pluronic® hydrogel), a porous silk scaffold, a plurality of microspheres, a lipososmal formulation and a hydroxyapatite fiber mesh. The carrier is an appropriate substrate for cells suited for ingrowth, proliferation and residence of IVD cells.

The carrier can comprise a polymer such as Pluronics e.g. pluronic 168, a block copolymer of ethylene oxide and propylene oxide such as those described in WO2005/034800.

Preferably, the carrier comprises chondroitin sulfate, gelatin, hyaluronan and/or sodium hyaluronate or a mixture thereof including tri-copolymers such as gelatin/chondroitin-6-sulfate/hyaluoran tri-copolymer. Preparations of tri-copolymers are described in Yang et al. (Yang et al., 2005).

The carrier may comprise a fibrin gel composed of platelet-rich plasma, platelet enriched plasma with biodegradable gelatin hydrogel, fibrin/hyaluronic acid composites, factor encapsulated gelatine hydrogel microspheres, injectable biodegradable hydrogel composites comprising for example polymers such as oligo(poly(ethyleneglycol) fumarate, polylactide (PLA) /polyglycolicacid (PGA) and poly epsilon capronolactone.

Preferably, the carrier comprises a chitosan-glycerol phosphate or an *in situ* blood clot or blood clot stabilized with chitosan-glycerol phosphate solution.

In one embodiment of the invention, the spinal nucleus pulposus implant further comprises a sustained release device e.g. a sustained release device comprising a hydrogel, polyanionic hydrogel, a plurality of microspheres, a liposomal formulation and a hydroxyapatite fiber mesh. The sustained release device, in particular, enables controlled release.

In one embodiment, the sustained release device provides continuous release, in another embodiment, the sustained release device provides intermittent release.

In one embodiment, the cartilage differentiation and maintenance factor is encapsulated in liposomes. The liposomes have the advantage over a crystalline solution in that a mechanical irritation in the intervertebral disc can be avoided and hence a therapy induced inflammation can be avoided in addition to a longer duration of the therapeutic agent and slower clearance at the site of application.

One approach for improving efficacy of delivery of therapeutic compounds and other agents has been the encapsulation in a lipid structure such as liposomes. Liposomes generally comprise an enclosed lipid droplet having a core typically containing a compound in an aqueous medium. In certain embodiments, the compound is chemically bound to a lipid component or simply contained within the aqueous inside compartment of the liposome.

A pharmaceutical composition or spinal nucleus pulposus implant provided according to the present invention comprising the cartilage differentiation and maintenance factor is preferably provided as dried liposomal compositions that can be reconstituted to produce liposomes encapsulating the cartilage differentiation and maintenance factor. Preferably, the liposomal preparation are dried reconstituted vesicles (DRVs) which upon reconstitution in an aqueous solution form cartilage differentiation and maintenance factor encapsulated liposomes. The liposomal composition used herein is for example a dry granular product which upon addition of water disperses to form multi-lamellar liposomal formulations comprising the biological active component. Advantageously, stability problems such as aggregation or oxidation of the active agent and/or liposomes are avoided by using dried liposomes.

Suitable lipids for use in the formulations which are present individually or in mixtures include neutral or positively charged lipids such as cholesterol, phosphatidylcholine, hydrogenated phosphatidylcholine, distearoylphosphatidylcholine, sphingomyelin, dioleyl phosphatidylcholine, dioleylphosphatidylglycerol, phosphatidylglycerol, dimyristoylphosphatidylcholine, dipamlitoylcholine, gangliosides, ceramides, phosphatidyinositol, phosphatic acids, dicetylphosphate, dimyrylstoyl phosphatidylcholine, stearylamine, dipalmitoyl phosphatidylgycerol and other similar lipids. Preferably the lipid mix is charged. The liposomal formulation is typically a mixture of at least two lipids such as cholesterol and phosphatidylcholine and more usually a mixture of three or more lipids.

In another embodiment, the cartilage differentiation and maintenance factor of the present invention is pegylated. This modified cartilage differentiation and maintenance factor has a biological half-life time greater than the unmodified agent and therefore can improve the efficacy of the agent for medical treatment of spinal disorders. Pegylation can increase the size of the protein, improve stability, increase solubility of the protein, reduce proteolysis and decrease dosing frequency. In addition, tendency towards aggregation of the protein can be reduced.

Pegylation can be achieved via stable covalent bonds between an amino or sulfhydryl group on the protein and a chemically reactive group (carbonate, ester, aldehyde, or tresylate) on the polyethylenglycol (PEG). The resulting structure may be linear or branched. PEG reagents are for example described in Roberts et al. (Roberts et al., 2002). Other pegylation agents are but are not limited to methoxypoly(ethylene glycol) (mPEG), methyl PEO₁₂ maleimide PEG, amine-reactive, methyl-capped polyethylene oxide (PEO)-containing modification agents (Methyl PEOₙ-NHS Esters, n=4, 8,12). In another embodiment, the spinal nucleus implant of the present invention further comprises nucleus pulposus tissue or cells, preferably cells derived from mesenchymal stem cells (MSCs).

MSCs or autologous stem cells isolated from the donor tissue (e.g. bone marrow stroma) can be cultured in or on a three-dimensional biodegradable scaffold material such as hyaluronic acid, silk, collagen, collagen/hyaluronan scaffolds, hydrogels, chitosan, chitosan gel, injectable cross-linkable polymeric preparations, degradable polymer gels or scaffolds, polylactide, gelatin/chondroitin-6-sulfate/hyaluronan scaffold, ester or derivatives of hyaluronic acid such as Hyaff 11, a hydroxyapatite fiber mesh and fibrin glue in the presence of a cartilage differentiation and maintenance factor either alone or in combination with other morphogens or growth factors like IGF-1 members or BMPs, which support the differentiation of those cells into nucleus pulposus-like cells and stimulate PG synthesis preferably under oxygen tension. Methods for cultivation are for example described in Honda et al., 2000, which are herewith incorporated by reference (Honda et al., 2004). The thus cultured cells or neotissue, preferably, along with the scaffold material, will then be transplanted or injected into the affected disc to achieve regeneration of the NP.

It is also possible to isolate and expand MSCs in monolayers culture for example under hypoxic conditions *in vitro,* as occurs in the NP region of the intervertebral disc. MSCs progressing to transplantation can be transfected with one or more cartilage differentiation and maintenance factor required to stimulate IVD healing or can be stimulated with a chondrogenic induction medium containing such cartilage differentiation and maintenance factor (e.g. CD-RAP or active fragments thereof). Preferably, a transfection can be performed by an expression vector encoding such cartilage differentiation and maintenance factor like the CD-RAP protein or active fragments thereof. Transfected and/or stimulated MSCs or cultured MSCs preferably embedded in a biomaterial such as collagen, atelocollagen gel, gelatine, alginate, hydroxypropylalginate, carboxymethylcellulose or hydroyethylstarch can be transplanted into the degenerative disc through injection such as an insulin microinjector (Sakai et al., 2005) or another application device. Cell density can be for example between 1 x 10⁴ cells/ml to 1 x 10⁷ cells/ml, preferably between 1 x 10⁵ cells/ml to 1 x 10⁶ cells/ml.

MSCs also can be cultured in alginate, pellet, micromass or aggregate cell cultures in the presence of the cartilage differentiation and maintenance factor either alone or in combination with other morphogens or growth factors like TGF-β members and/or BMPs (e.g. BMP-2), which support the differentiation of those cells into NP-like cells.

In one embodiment, the delivery device further comprises MSCs or AF cells in combination with a 3-D porous silk scaffold. The porous silk scaffold may be derived from silk fibroin extracted from Bombyx mori. The cells can be expanded undifferentiated and will be induced into chondrocytes by culturing with the cartilage differentiation and maintenance factor. Silk scaffolds can either directly or loaded with the cartilage differentiation and maintenance factor be seeded with MSCs or AF cells and can be cultured in medium supplemented with or without the cartilage differentiation and maintenance factor alone or in combination with other factors described above. Silk fibroin scaffolds from Bombyx mori silkworm cocoons can be extracted as for example described in Sofia et al. and Karageorgiou et al. (Sofia et al., 2001; Karageorgiou and Kaplan, 2005).

In one embodiment, AF, NP or MSC cells can be transfected *ex vivo* with at least one gene for a cartilage differentiation and maintenance factor to provide both the cells and the protein or proteins required to stimulate IVD healing and are reimplanted with or without culturing in for example monolayer cultures, alginate beads or a three-dimensional biodegradable scaffold material into the targeted host tissue. As an example, isolated NP cells are seeded as monolayer, followed by transfection with the cartilage differentiation and maintenance factor comprising for example an expression vector or viral gene therapy vector such as the adeno-virus or adeno-associated virus (AAV) using a transfection agent such as the FuGene6 reagent. After several days of culture (e.g. 7 days), cells are passaged and encapsulated in alginate e.g. preferably between about 0,5 to 2% alginate. Expression of the gene can be analyzed by standard methods such as RT-PCR or ELISA. These transfected nucleus pulposus cells in alginate beads can be used for tissue engineering of the IVD and treatment for the degenerative disc disease. Further gene therapy methods are described for example in Wells, 2004; Paul et al., 2003 and Sobajima et al., 2004.

In one embodiment cells such as NF cells, MSCs or autologous chondrocytes preferably of human origin are temporarily immortalized using for example a recombinant Simian Virus 40 adenovirus vector or baculovirus vector encoding for the cartilage differentiation and maintenance factor required to stimulate IVD healing.

In another embodiment degenerate human IVD cells (e.g. NP cells) can be transfected with an adenovirus vector carrying the exogenous factor such as the cartilage differentiation and maintenance factor. A subsequent step would then be to inject these modified cells back into the diseased IVD.

It is also disclosed the use of a cartilage differentiation and maintenance factor of the present invention for culturing mesenchymal stem cells for manufacturing of a pharmaceutical composition for treating a spinal disorder in a mammal, in particular, in a human in need of such treatment.

The invention is further illustrated by the Figures and Examples.
**FIGURE 1** illustrates the stability of the liposomal formulation comprising CD-RAP over several days (triangles). Stability of the liposomal formulation was determined according to example 3. The upper curve (squares) shows the stability of CD-RAP in buffer at 37°C to determine stability of the protein under these conditions.
**FIGURE 2** shows the immobilization of 50 *µ*g CD-RAP in the fibrin clot system after 24 h at 37 °C.
   A: Tachotop®, 14 mm diameter, 4 mm height from Nycomed; B: porcine type I collagen sponge, BiomUp; C: rehydrated Hyalofill-F.
**FIGURE 3** illustrates the percent (%) proteoglycan (GAG) induction of cultured rabbit IVD cells upon stimulation with CD-RAP (100 ng/ml) related to the negative control without addition of CD-RAP and BMP-2 (n=3).

### Examples

### Example 1: Annulus fibrosus puncture mode!

In this example, an injection of CD-RAP is effective in partially restoring the disc height in a rabbit annular puncture model.

Disc degeneration can be induced in adolescent New Zealand White Rabbits by puncture of the annulus fibrosus of the disc using defined needle gauges (Singh et al., 2005). After provision of a local anaesthetic by injection of lidocain to the dorsal region of the disc lateral plain radiographs are obtained to determine preinjection baseline values for IVD heights. Subsequently the rabbits are placed into a lateral prone position and a posterolateral retroperitoneal approach is used to expose the lumbar IVDs. In each rabbit the AF will be punctured with an 18G needle. After four, eight and 10 weeks the animal receive an injection of buffered saline (arginine phosphate or PBS) or vehicle liposomes as a control or protein solution of 2.5 mg/ml to 10 mg/ml CD-RAP (in PBS) or liposomal encapsulated CD/RAP (2.5 mg/ml) into the nucleus pulposus. The animals are followed for 8 or 12 weeks (induction and treatment period). Preclinical outcome is analyzed by magnetic resonance imaging (MRI) scans of the lumbar spine, IVD height is monitored by radiological observation measured with a custom program using Imaging software and the %DHI (postoperative DHI/preoperative DHIx100) is calculated. For histological analysis of the IVDs, sections are stained with Hematoxylin Eosin and Safranin O. Differences among groups are assessed for statistical significance by using a one-way analysis of variance (ANOVA). An alternative slow progressive and reproducible animal model of disc degeneration therapies is a classical "stab model" of Lipson and Muir as described in Sobajima et al. (Sobajima et al., 2005). For the stab method, an incision will be made in an AF of New Zealand White rabbits. Each rabbit will have one disc treated with CD-RAP, the other with saline solution or vehicle.

The annulus needle puncture model results in disc narrowing with the 12 week observation period. Saline treated discs exhibit extensive degeneration of the disc. However, the %DHI reveals a tendency of a preserved disc height in the CD-RAP group compared to the saline or vehicle injected discs. Histological analysis shows an increase of proteoglycan synthesis and protection against degenerative changes compared to the control group.

### Example 2: Preparation of CD/RAP containing liposomes

For preparation of a sustained liposomal preparation 750 mg phosphatidylcholine and 250 mg cholesterol were solved in 20 ml ethanol in a round bottom flask. The solvent was removed in a rotary evaporator quantitatively. The generated thin lipid film was rehydrated in 10 ml water to get liposomes (10 % (w/v) lipid) by gentle stirring at room temperature. Unilamellar vesicles (SUV) were prepared with a diameter of approximately 100 nm by subsequent sonification. 300 *µ*l of SUV were mixed with 250 *µ*l CD-RAP solution (3 mg/ml in 420 mM/l Arg/PO4 pH 7.5) and were subsequently lyophilized. Multilamellar liposomes (MLVs) encapsulating the protein were generated by reconstitution of the lyo cake with destilled water shortly before application of the dried reconstituted vesicles. This rehydration led to an entrapment efficacy of about 50 % or more into MLVs with an average diameter of about 1.5 *µ*m or more without chemical alteration of the entrapped drug.

### Example 3: Stability of liposomal formulation comprising CD-RAP

This example illustrates the stability of the liposomal formulation comprising CD-RAP over several days. Stability of the liposomal formulation was determined as follows:
120 *µ*g of CD-RAP were encapsulated in 300 *µ*l liposomal suspension as described above. An aliquot of 100 *µ*l was diluted with 300 *µ*l bidestilled water and separated by centrifugation for 15 min at 16000 rcf. To determine the encapsulation efficacy, the non-encapsulated CD-RAP was quantified by RP-HPLC using a standard curve. A six-fold repetition of a resuspension and centrifugation step did not show increase of the free CD-RAP.

The release was described by measuring the free concentration of CD-RAP in the supernatant within a time period of seven days, separated by centrifugation (15 min at 16000 rcf). At each time point the amount of free CD-RAP was measured by RP-HPLC using a standard curve.

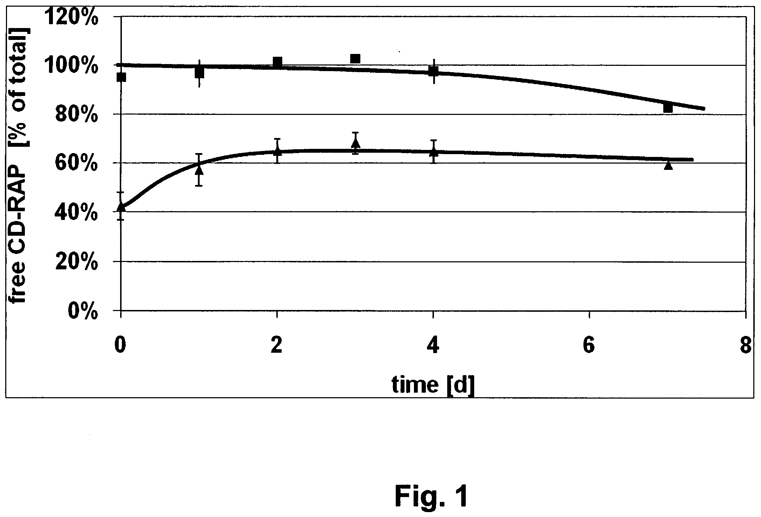

The results shown in Fig. 1 indicate a high encapsulation efficacy of more than 50% (time 0 [d]). During the following observation time period of up to 7 days at 37°C a plateau was achieved with maintenance of an encapsulation of about 45%, therefore indicating stability of the reconstituted CD-RAP comprising liposomes.

### Example 4: CD-RAP immobilized implants

This example illustrates CD-RAP immobilized implants in collagen or hyaluronic acid based scaffolds.
50 *µ*g of CD-RAP were formulated in 125 *µ*l 20 mM KH₂PO4, 150 mM KCI, KOH, pH 7.5, 0.01 % Tween 80. The solution was soaked into a collagen sponge (A: Tachotop® (A), 14 mm diameter, 4 mm height from Nycomed; (B) porcine type I collagen sponge, BiomUp; or mixed with 500 *µ*l Hyaff gel (rehydrated Hyalofill-F (C), 30 mg in 0.5 ml bidest water, 3 h, 5 °C) to adsorb CD-RAP. Subsequently the water was removed by freeze drying.

To determine the immobilization kinetic of CD-RAP adsorbed onto collagen or Hyaff, the CD-RAP impregnated specimen was fixed within 500 *µ*l bovine fibrin clot (4.9 mg fibrinogen, 0.3 U thrombin in 50 mM sodium citrate, 150 mM sodium chloride, 10 mM calcium chloride, pH 6.4) as described by Meyenburg et al. (Meyenburg et al., 2000). The clot was then covered completely by 2 ml acceptor medium (phosphate buffered saline, 0.02 % Tween 80). The amount of free CD-RAP was quantified after 24 h by RP-HPLC using a standard curve.

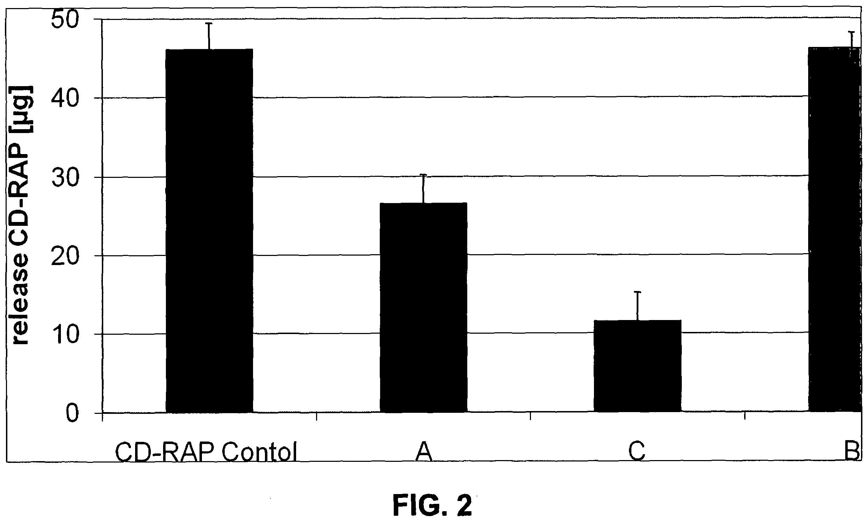

The results shown in Fig. 2 indicate differences in immobilization efficacy of various biomaterials with material A and C showing strong binding properties for the recombinant CD-RAP protein. These binding properties can be used for local targeting and retention of the cartilage determination and maintenance factor within the site of defect e.g. the degenerated disc avoiding a high initial burst of CD-RAP and providing long-term maintenance at the site of regeneration.

### Example 5: Isolation of human and animal intervertebral disc cells

This example illustrates methods to prepare IVD cells useful for analyzing the effect of CD-RAP on production of extracellular matrix components specific for cartilage cell stimulation and anabolic activity of CD-RAP.

Human disc cells can be isolated from human disc tissue recovered by disectomy performed in the treatment of patients with degenerative disc disease. The specimen (nucleus pulposus or annulus fibrosus) are rinsed with PBS to remove residual blood or extracellular matrix. After mincing of the tissue cells are released from the extracellular matrix with collagenase solution (0,5 mg/ml in PBS) at 37°C for 45 min and cells can be isolated by centrifugation (see Klagsburn, "Methods in Enzymology", Vol VII). After removal of the supernatant harvested cells are grown on six well plates in Eagle minimal essential medium with or without fetal calf serum.

Bovine IVD cells from animal tissue are isolated by sequential enzymatic digestion. Cells are cultivated with daily medium changes of DMEM/F12 medium supplemented with 10% fetal bovine serum, 25 µg/ml ascorbate, 360 µg/ml L-glutamine and 50 µg/ml gentamicin in a humidified atmosphere at 37°C with 5% CO₂ until they have reached about 80% confluence.

Rabbit IVD cells from animal tissue were isolated by sequential enzymatic digestion. Cells were cultivated in DMEM medium supplemented with 10% FCS, 1% Penicillin/Streptavidin and 50ng/ml ascorbic acid in a humidified atmosphere at 37°C with 5% CO₂ until they reached about 90% confluence. Thereafter they were passaged and cultivated for additional 7 days.

### Example 6: Culturing of intervertebral disc cells in alginate beads

Alginate beads are formed by expressing 60 µl 1,2% alginate in 0,15 NaCl with IVD cells of example 5 into a 102 mmol/L calcium chloride solution, forming a semisolid bead. The beads are washed twice and placed in 12 well plates with 1 ml medium (Aota et al., 2005). The resulting alginate beads can be used for analyzing the influence of fibronectin fragments (120 kDa fragment (Chemicon, Cat. No. F1904), 70 kDa fibronectin proteolytic fragment from human plasma (Sigma, Cat. No. F0287)) with or without addition of CD-RAP for proteoglycan synthesis and aggrecan expression.

### Example 7: CD-RAP mediated induction of aggrecan and proteoglycan synthesis in IVD cells

This example illustrates the use of the spinal implant to demonstrate that CD-RAP when added to IVD cells significantly increases proteoglycan synthesis and aggrecan expression of IVD cell during culturing.

On day 7 IVD cells of example 5 are placed in 24 well plates to study proteoglycan synthesis and expression of aggrecan. Cells are cultured for 7 more days in serum free medium with 0.5 *µ*M and 0.1 *µ*M of a 70 kDa fragment of fibronectin (FO297 Sigma). For analyzing the influence of CD-RAP on proteoglycan synthesis and expression of aggrecan different concentrations of CD-RAP (1, 5, 10, 20 ug/ml) are added two days after culturing with fibronectin. After 7 days aggrecan expression is analyzed by Lightcycler analysis (SybrGreen).

To release the cells from the beads, the bead are solubilized in a buffer containing 55 mmol/L sodium citrate, 30 mmol/L Na₂EDTA, 0,15M sodium chloride pH 6,8. The cell pellets are washed and resuspended in lysis buffer for RNA extraction (Qiagen). RNA isolation is performed using the RNeasy Mini Kit (Qiagen). CDNA synthesis is performed according to the instruction of the Superscript II RT kit of Invitrogen. β-actin is used as a control. Primers used for amplification are the following: a) bovine β-actin primers 5' GGA AAT CGT CCG TGA CAT CAA 3'; 5' AAG GAA GGC TGG AAG AGA GC 3'; Aggrecan primers were: 5' AAG AGA GCC AAA CAG CCG A 3'; 5' CTG GTA GTC CTG GGC ATT GT 3'.

Proteoglycan synthesis is measured using the dimethylmethylene blue (DMMB) colorimetric assay according to the method as described in Farndale et al. (Farndale et al., 1982). The culture medium can be concentrated with a centricon filter for 10 to 20 minutes at 5000 rev/min. The GAG content can be determined by mixing 20 *µ*l of concentrated or diluted culture medium with 200 *µ*l of DMMB solution and measuring the optical density at 525 - 530 nm. For standardization chondroitin sulfate (chondroitinsulfat A 94%, bovine trachea, ICN) can be used. The mean of all measures are calculated per microgram of DNA or cell number.

### Example 8: Proteoglycan induction in rabbit intervertebral disc cells

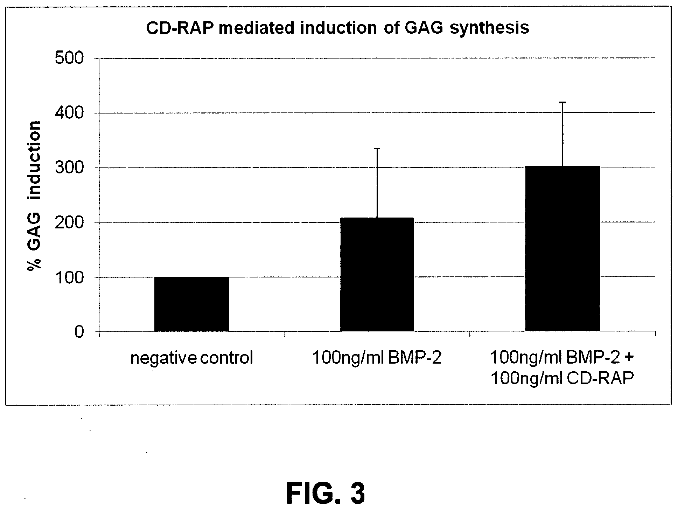

When rabbit IVD cells isolated according to example 5 reached 90% confluence cells were placed in 6 well plates to study proteoglycan synthesis. Cells were cultured for 5 more days. For analyzing the influence of CD-RAP on proteoglycan synthesis the medium was changed to 1% FCS and BMP-2 (100 ng/ml) alone or in combination with CD-RAP (100ng/ml) was added to the culture. After 5 days proteoglycan synthesis was measured in the cell culture supernatant using the dimethylmethylene blue (DMMB) colorimetric assay as described in example 7. The results of GAG stimulation of three independent experiments are summarized in Fig.3. These data indicate that the addition of CD-RAP to BMP-2 stimulated IVD cells led to an increase of GAG in cultured rabbit IVD cells.

### Reference List

Aota,Y., An,H.S., Homandberg,G., Thonar,E.J., Andersson,G.B., Pichika,R., and Masuda,K. (2005). Differential effects of fibronectin fragment on proteoglycan metabolism by intervertebral disc cells: a comparison with articular chondrocytes. Spine 30, 722-728.
Bauer,R., Humphries,M., Fassler,R., Winklmeier,A., Craig,S.E., and Bosserhoff,A.K. (2006). Regulation of integrin activity by MIA. J Biol Chem 281, 11669-11677.
Bosserhoff,A.K. and Buettner,R. (2003). Establishing the protein MIA (melanoma inhibitory activity) as a marker for chondrocyte differentiation. Biomaterials 24, 3229-3234.
Bosserhoff,A.K., Kondo,S., Moser,M., Dietz,U.H., Copeland,N.G., Gilbert,D.J., Jenkins,N.A., Buettner,R., and Sandell,L.J. (1997). Mouse CD-RAP/MIA gene: structure, chromosomal localization, and expression in cartilage and chondrosarcoma. Dev. Dyn. 208, 516-525.
Bosserhoff,A.K., Moser,M., and Buettner,R. (2004). Characterization and expression pattern of the novel MIA homolog TANGO. Gene Expr. Patterns. 4, 473-479.
Dietz,U.H. and Sandell,L.J. (1996). Cloning of a retinoic acid-sensitive mRNA expressed in cartilage and during chondrogenesis. J. Biol. Chem. 271, 3311-3316.
Farndale,R.W., Sayers,C.A., and Barrett,A.J. (1982). A direct spectrophotometric microassay for sulfated glycosaminoglycans in cartilage cultures. Connect. Tissue Res. 9, 247-248.
Homandberg,G.A. and Hui,F. (1996). Association of proteoglycan degradation with catabolic cytokine and stromelysin release from cartilage cultured with fibronectin fragments. Arch. Biochem. Biophys. 334, 325-331.
Homandberg,G.A., Hui,F., Wen,C., Purple,C., Bewsey,K., Koepp,H., Huch,K., and Harris,A. (1997). Fibronectin-fragment-induced cartilage chondrolysis is associated with release of catabolic cytokines. Biochem. J 321 (Pt 3), 751-757.
Honda,M.J., Yada,T., Ueda,M., and Kimata,K. (2004). Cartilage formation by serial passaged cultured chondrocytes in a new scaffold: hybrid 75:25 poly(L-lactide-epsilon-caprolactone) sponge. J Oral Maxillofac Surg 62, 1510-1516.
Karageorgiou,V. and Kaplan,D. (2005). Porosity of 3D biomaterial scaffolds and osteogenesis. Biomaterials 26, 5474-5491.
Kawakami,M., Matsumoto,T., Hashizume,H., Kuribayashi,K., Chubinskaya,S., and Yoshida,M. (2005). Osteogenic protein-1 (osteogenic protein-1/bone morphogenetic protein-7) inhibits degeneration and pain-related behavior induced by chronically compressed nucleus pulposus in the rat. Spine 30, 1933-1939.
Kelley,L.A., MacCallum,R.M., and Sternberg,M.J. (2000). Enhanced genome annotation using structural profiles in the program 3D-PSSM. J Mol. Biol 299, 499-520.
Levicoff,E.A., Gilbertson,L.G., and Kang,J.D. (2005). Gene therapy for disc repair. Spine J 5, 287S-296S.
Lougheed,J.C., Holton,J.M., Alber,T., Bazan,J.F., and Handel,T.M. (2001). Structure of melanoma inhibitory activity protein, a member of a recently identified family of secreted proteins. Proc. Natl. Acad. Sci. U. S. A 98, 5515-5520.
Masuda,K. and An,H.S. (2004). Growth factors and the intervertebral disc. Spine J 4, 330S-340S.
Masuda,K., Oegema,T.R., Jr., and An,H.S. (2004). Growth factors and treatment of intervertebral disc degeneration. Spine 29, 2757-2769.
Meyenburg,S., Lilie,H., Panzner,S., and Rudolph,R. (2000). Fibrin encapsulated liposomes as protein delivery system. Studies on the in vitro release behavior. J Control Release 69, 159-168.
Paul,R., Haydon,R.C., Cheng,H., Ishikawa,A., Nenadovich,N., Jiang,W., Zhou,L., Breyer,B., Feng,T., Gupta,P., He,T.C., and Phillips,F.M. (2003). Potential use of Sox9 gene therapy for intervertebral degenerative disc disease. Spine 28, 755-763.
Roberts,M.J., Bentley,M.D., and Harris,J.M. (2002). Chemistry for peptide and protein PEGylation. Adv. Drug Deliv. Rev 54, 459-476.
Saito,S., Kondo,S., Mishima,S., Ishiguro,N., Hasegawa,Y., Sandell,L.J., and Iwata,H. (2002). Analysis of cartilage-derived retinoic-acid-sensitive protein (CD-RAP) in synovial fluid from patients with osteoarthritis and rheumatoid arthritis. J Bone Joint Surg Br 84, 1066-1069.
Sakai,D., Mochida,J., Iwashina,T., Watanabe,T., Nakai,T., Ando,K., and Hotta,T. (2005). Differentiation of mesenchymal stem cells transplanted to a rabbit degenerative disc model: potential and limitations for stem cell therapy in disc regeneration. Spine 30, 2379-2387.
Sakano,S., Zhu,Y., and Sandell,L.J. (1999). Cartilage-derived retinoic acid-sensitive protein and type II collagen expression during fracture healing are potential targets for Sox9 regulation. J. Bone Miner. Res. 14, 1891-1901.
Singh,K., Masuda,K., and An,H.S. (2005). Animal models for human disc degeneration. Spine J 5, 267S-279S.
Sobajima,S., Kim,J.S., Gilbertson,L.G., and Kang,J.D. (2004). Gene therapy for degenerative disc disease. Gene Ther. 11, 390-401.
Sobajima,S., Kompel,J.F., Kim,J.S., Wallach,C.J., Robertson,D.D., Vogt,M.T., Kang,J.D., and Gilbertson,L.G. (2005). A slowly progressive and reproducible animal model of intervertebral disc degeneration characterized by MRI, X-ray, and histology. Spine 30, 15-24.
Sofia,S., McCarthy,M.B., Gronowicz,G., and Kaplan,D.L. (2001). Functionalized silk-based biomaterials for bone formation. J Biomed Mater Res 54, 139-148.
Stoll,R., Renner,C., Buettner.R., Voelter,W., Bosserhoff,A.K., and Holak,T.A. (2003). Backbone dynamics of the human MIA protein studied by (15)N NMR relaxation: implications for extended interactions of SH3 domains. Protein Sci. 12, 510-519.
Stoll,R., Renner,C., Zweckstetter,M., Bruggert,M., Ambrosius,D., Palme,S., Engh,R.A., Golob,M., Breibach,l., Buettner,R., Voelter,W., Holak,T.A., and Bosserhoff,A.K. (2001a). The extracellular human melanoma inhibitory activity (MIA) protein adopts an SH3 domain-like fold. EMBO J. 20, 340-349.
Stoll,R., Renner,C., Zweckstetter,M., Bruggert,M., Ambrosius,D., Palme,S., Engh,R.A., Golob,M., Breibach,l., Buettner,R., Voelter,W., Holak,T.A., and Bosserhoff,A.K. (2001b). The extracellular human melanoma inhibitory activity (MIA) protein adopts an SH3 domain-like fold. Embo J 20, 340-349.
Takegami,K., An,H.S., Kumano,F., Chiba,K., Thonar,E.J., Singh,K., and Masuda,K. (2005). Osteogenic protein-1 is most effective in stimulating nucleus pulposus and annulus fibrosus cells to repair their matrix after chondroitinase ABC-induced in vitro chemonucleolysis. Spine J 5, 231-238.
Tscheudschilsuren,G., Bosserhoff,A.K., Schlegel,J., Vollmer,D., Anton,A., Schnettler,R., Brandt,J., and Proetzel,G. (2005). Regulation of mesenchymal stem cell and chondrocyte differentiation by MIA. Experimental Cell Research 1-10.
Wells,D.J. (2004). Gene therapy progress and prospects: electroporation and other physical methods. Gene Ther. 11, 1363-1369.
Wozney,J.M. and Rosen,V. (1998). Bone morphogenetic protein and bone morphogenetic protein gene family in bone formation and repair. Clin Orthop 346, 26-37.
Yang,S.H., Chen,P.Q., Chen,Y.F., and Lin,F.H. (2005). An in-vitro study on regeneration of human nucleus pulposus by using gelatin/chondroitin-6-sulfate/hyaluronan tri-copolymer scaffold. Artif. Organs 29, 806-814.

## Claims

1. A spinal nucleus pulposus implant or formulation comprising a cartilage differentiation and maintenance factor comprising the mature sequence of CD-RAP according to Seq. ID No. 1, or amino acids 12 to 107 of Seq. ID No. 1, for use in the treatment of a spinal disorder.

2. The spinal nucleus pulposus implant or formulation for use according to claim 1, wherein the implant is injectable or implantable transdiscally.

3. The spinal nucleus pulposus implant or formulation for use according to claim 1 or 2, wherein the implant or formulation comprises a carrier.

4. The spinal nucleus pulposus implant or formulation for use according to any of claims 1 to 3, wherein the implant or formulation comprises a sustained release device.

5. The spinal nucleus pulposus implant or formulation for use according to any of claims 1 to 4, wherein the cartilage differentiation and maintenance factor is encapsulated in liposomes.

6. The spinal nucleus pulposus implant or formulation for use according to any of claims 1 to 5, wherein the spinal nucleus pulposus implant comprises nucleus pulposus tissue or cells.

7. Use of a cartilage differentiation and maintenance factor comprising the mature sequence of CD-RAP according to Seq. ID No. 1, or amino acids 12 to 107 of Seq. ID No. 1, for the manufacture of a pharmaceutical composition, which is a spinal nucleus pulposus implant or formulation, for the treatment of a spinal disorder.

8. Use of claim 7, wherein the spinal disorder is idiopathic low back pain, disc herniation, internal disc disruption or fissured discs, radiculopathy, spinal stenosis, herniated nucleus pulposus-induced sciatica, sciatica, idiopathic scoliosis or myelopathy.

9. Use of claim 7 or 8, wherein the pharmaceutical composition has the features as defined in any of claims 3 to 6.

10. The spinal nucleus pulposus implant or formulation for use according to claim 1, wherein the spinal disorder is idiopathic low back pain, disc herniation, internal disc disruption or fissured discs, radiculopathy, spinal stenosis, herniated nucleus pulposus-induced sciatica, sciatica, idiopathic scoliosis or myelopathy.

## Patentansprüche

1. Nucleus Pulposus-Spinalimplantat oder -Formulierung, umfassend einen Knorpel-Differenzierungs- und einen Erhaltungsfaktor, umfassend die reife Sequenz von CD-RAP entsprechend Seq. ID Nr. 1 oder die Aminosäuren 12 bis 107 von Seq. ID Nr. 1, zur Verwendung bei der Behandlung einer Wirbelsäulenerkrankung.

2. Nucleus-Pulposus-Spinalimplantat oder -Formulierung zur Verwendung nach Anspruch 1, wobei das Implantat injizierbar oder transdiskal implantierbar ist.

3. Nucleus-Pulposus-Spinalimplantat oder -Formulierung zur Verwendung nach Anspruch 1 oder 2, wobei das Implantat oder die Formulierung einen Träger umfasst.

4. Nucleus-Pulposus-Spinalimplantat oder -Formulierung zur Verwendung nach einem der Ansprüche 1 bis 3, wobei das Implantat oder die Formulierung eine Vorrichtung mit verzögerter Freisetzung umfasst.

5. Nucleus-Pulposus-Spinalimplantat oder -Formulierung zur Verwendung nach einem der Ansprüche 1 bis 4, wobei der Knorpel-Differenzierungs- und -Erhaltungsfaktor in Liposomen eingekapselt ist.

6. Nucleus-Pulposus-Spinalimplantat oder -Formulierung zur Verwendung nach einem der Ansprüche 1 bis 5, wobei das Nucleus Pulposus-Spinalimplantat Nucleus Pulposus-Gewebe oder -Zellen umfasst.

7. Verwendung eines Knorpel-Differenzierungs- und Erhaltungsfaktors, umfassend die reife Sequenz von CD-RAP gemäß Seq. ID Nr. 1 oder die Aminosäuren 12 bis 107 von Seq. ID Nr. 1, zur Herstellung einer pharmazeutischen Zusammensetzung, die ein Nucleus Pulposus-Spinalimplantat oder eine Nucleus Pulposus-Formulierung ist, zur Behandlung einer Wirbelsäulenerkrankung.

8. Verwendung nach Anspruch 7, wobei die Wirbelsäulenerkrankung idiopathischer Rückenschmerz, Bandscheibenvorfall, innere Diskusruptur oder zerklüftete Bandscheiben, Radikulopathie, spinale Stenose, Diskusprolaps-induzierter Ischias, Ischias, idiopathische Skoliose oder Myelopathie ist.

9. Verwendung nach Anspruch 7 oder 8, wobei die pharmazeutische Zusammensetzung die in einem der Ansprüche 3 bis 6 definierten Merkmale aufweist.

10. Nucleus-Pulposus-Spinalimplantat oder -Formulierung zur Verwendung nach Anspruch 1, wobei die Wirbelsäulenerkrankung idiopathischer Rückenschmerz, Bandscheibenvorfall, innere Diskusruptur oder zerklüftete Bandscheiben, Radikulopathie, spinale Stenose, Diskusprolaps-induzierter Ischias, Ischias, idiopathische Skoliose oder Myelopathie ist.

## Revendications

1. Implant ou formulation pour nucleus pulposus spinal comprenant un facteur de différenciation et d'entretien du cartilage comprenant la séquence mature de CD-RAP selon Seq. ID No. 1, ou les acides aminés 12 à 107 de Seq. ID No. 1, destiné à être utilisé dans le traitement d'un trouble spinal.

2. Implant ou formulation pour nucleus pulposus spinal destiné à être utilisé selon la revendication 1, où l'implant est injectable ou implantable de manière transdiscale.

3. Implant ou formulation pour nucleus pulposus spinal destiné à être utilisé selon la revendication 1 ou 2, où l'implant ou formulation comprend un vecteur.

4. Implant ou formulation pour nucleus pulposus spinal destiné à être utilisé selon l'une quelconque des revendications 1 à 3, où l'implant ou formulation comprend un dispositif pour libération prolongée.

5. Implant ou formulation pour nucleus pulposus spinal destiné à être utilisé selon l'une quelconque des revendications 1 à 4, où le facteur de différenciation et d'entretien du cartilage est encapsulé dans des liposomes.

6. Implant ou formulation pour nucleus pulposus spinal destiné à être utilisé selon l'une quelconque des revendications 1 à 5, où l'implant pour nucleus pulposus spinal comprend du tissu ou des cellules de nucleus pulposus.

7. Utilisation d'un facteur de différenciation et d'entretien du cartilage comprenant la séquence mature de CD-RAP selon Seq. ID No. 1, ou les acides aminés 12 à 107 de Seq. ID No. 1, pour la fabrication d'une composition pharmaceutique, qui est un implant ou formulation pour nucleus pulposus spinal, pour le traitement d'un trouble spinal.

8. Utilisation selon la revendication 7, où le trouble spinal est la douleur du bas du dos idiopathique, la hernie discale, la rupture discale interne ou les disques fissurés, la radiculopathie, la sténose spinale, la sciatique induite par nucleus pulposus hernié, la sciatique, la scoliose idiopathique ou la myélopathie.

9. Utilisation selon la revendication 7 ou 8, où la composition pharmaceutique a les caractéristiques telles que définies dans l'une quelconque des revendications 3 à 6.

10. Implant ou formulation pour nucleus pulposus spinal destiné à être utilisé selon la revendication 1, où le trouble spinal est la douleur du bas du dos idiopathique, la hernie discale, la rupture discale interne ou les disques fissurés, la radiculopathie, la sténose spinale, la sciatique induite par nucleus pulposus hernié, la sciatique, la scoliose idiopathique ou la myélopathie.


### SEQUENCE LISTING

<110> Scil Technology GmbH
<120> A spinal nucleus pulposus implant
<130> 38961PCT
<150> EP 06021093.7
   <151> 2006-10-06
<160> 1
<170> PatentIn version 3.3
<210> 1
   <211> 107
   <212> PRT
   <213> Homo sapiens
<400> 1
   Gly Pro Met Pro Lys Leu Ala Asp Arg Lys Leu Cys Ala Asp Gln Glu
   1 5 10 15
   Cys Ser His Pro Ile Ser Met Ala Val Ala Leu Gln Asp Tyr Met Ala
   20 25 30
   Pro Asp Cys Arg Phe Leu Thr Ile His Arg Gly Gln Val Val Tyr Val
   35 40 45
   Phe Ser Lys Leu Lys Gly Arg Gly Arg Leu Phe Trp Gly Gly Ser Val
   50 55 60
   Gln Gly Asp Tyr Tyr Gly Asp Leu Ala Ala Arg Leu Gly Tyr Phe Pro
   65 70 75 80
   Ser Ser Ile Val Arg Glu Asp Gln Thr Leu Lys Pro Gly Lys Val Asp
   85 90 95
   Val Lys Thr Asp Lys Trp Asp Phe Tyr Cys Gln
   100 105